# EUROPEAN PATENT APPLICATION

(11) **EP 3 943 594 A1**
(43) Date of publication of application: **26.01.2022**
(21) Application number: 20925001.8
(22) Date of filing: 18.12.2020
(51) Int. Cl.: C12N 1/21, C12N 9/06, C12N 9/02, C12N 9/90, C12N 9/12, C12N 15/53, C12N 15/77, C12P 13/08

(54) **RECOMBINANT MICROORGANISM FOR PRODUCING L-VALINE, A CONSTRUCTION METHOD THEREFOR, AND APPLICATION THEREOF**

(30) Priority: 27.05.2020 CN 202010466347
(71) Applicant: Anhui Huaheng Biotechnology Co., Ltd., Suangfeng Industrial Park Hefei, Anhui 21131 (CN); Tianjin Institute Of Industrial Biotechnology, Chinese Academy of Sciences, Tianjin 300308 (CN)
(72) Inventor: ZHANG, Xueli, Tianjin 300308 (CN); GUO, Henghua, Hefei, Anhui 231131 (CN); LIU, Pingping, Tianjin 300308 (CN); ZHANG, Dongzhu, Hefei, Anhui 231131 (CN); TANG, Jinlei, Tianjin 300308 (CN); HAN, Chengxiu, Bayannaoer, Inner Mongolia 015000 (CN); TANG, Siqing, Bayannaoer, Inner Mongolia 015000 (CN); LIU, Shupeng, Hefei, Anhui 231131 (CN); MA, Yanhe, Tianjin 300308 (CN)
(74) Representative: Biggi, Cristina
(86) International application number: PCT/CN2020/137778
(87) International publication number: WO 2021/238183

(57) **Abstract**

Related are a recombinant microorganism for producing L-valine, a construction method and an application thereof. Through transferring an amino acid dehydrogenase gene and/or activating activity of a transhydrogenase and/or a NAD kinase, reducing power of NADPH in cell is increased, the titer and yield of L-valine generated by *Escherichia coli* are improved, and the production of L-valine by one-step anaerobic fermentation is achieved.

SEQUENCE LISTING

<110>  Anhui Huaheng Biological Engineering Co., Ltd.
       Tianjin Institute of Industrial Biotechnology, Chinese Academy of
Sciences

<120>  Recombinant microorganism for producing L-valine, construction method
and application thereof

<130>  PN151378HHSW

<150>  CN202010466347.0
<151>  2020-05-27

<160>  79

<170>  PatentIn version 3.5

<210>  1
<211>  73
<212>  DNA
<213>  Escherichia coli

<400>  1
gtaggaaagt taactacgga tgtacattat ggaactgacg actcgcactt tgtgacggaa      60

gatcacttcg cag                                                          73


<210>  2
<211>  75
<212>  DNA
<213>  Escherichia coli

<400>  2
gcgtttgcca cctgtgcaat attacttcag acggtccgcg agataacgct ttatttgtta      60

actgttaatt gtcct                                                        75


<210>  3
<211>  20
<212>  DNA
<213>  Escherichia coli

<400>  3
cagctcatca accaggtcaa                                                   20


<210>  4
<211>  20
<212>  DNA
<213>  Escherichia coli

<400>  4
aaaagccgtc acgttattgg                                                   20


<210>  5
<211>  70
<212>  DNA
<213>  Escherichia coli

<400>  5
gcgtttgcca cctgtgcaat attacttcag acggtccgcg agataacgct aagtgcgagt      60

cgtcagttcc                                                              70


<210>  6
<211>  72
<212>  DNA
<213>  Escherichia coli

<400>  6
gtaggaaagt taactacgga tgtacattat ggaactgacg actcgcactt atggctaact      60

acttcaatac ac                                                           72


<210>  7
<211>  75
<212>  DNA
<213>  Escherichia coli

<400>  7
gcgtttgcca cctgtgcaat attacttcag acggtccgcg agataacgct ttaacccgca      60

acagcaatac gtttc                                                        75


<210>  8
<211>  73
<212>  DNA
<213>  Escherichia coli

<400>  8
gtaggaaagt taactacgga tgtacattat ggaactgacg actcgcactt tgtgacggaa      60

gatcacttcg cag                                                          73


<210>  9
<211>  75
<212>  DNA
<213>  Escherichia coli

<400>  9
agctgtgcca gctgctggcg cagattcagt gtattgaagt agttagccat ttatttgtta      60

actgttaatt gtcct                                                        75


<210>  10
<211>  70
<212>  DNA
<213>  Escherichia coli

<400>  10
gtaggaaagt taactacgga tgtacattat ggaactgacg actcgcactt ttatctctgg      60

cggtgttgac                                                              70


<210>  11
<211>  72

<212>  DNA
<213>  Escherichia coli

<400>  11
agctgtgcca gctgctggcg cagattcagt gtattgaagt agttagccat agctgtttcc      60

tggtttaaac cg                                                          72


<210>  12
<211>  20
<212>  DNA
<213>  Escherichia coli

<400>  12
cgcactacat cagagtgctg                                                  20


<210>  13
<211>  73
<212>  DNA
<213>  Escherichia coli

<400>  13
ttcaacatca ctggagaaag tcttatgaaa ctcgccgttt atagcacaaa tgtgacggaa      60

gatcacttcg cag                                                         73


<210>  14
<211>  75
<212>  DNA
<213>  Escherichia coli

<400>  14
agcggcaaga ttaaaccagt tcgttcgggc aggtttcgcc tttttccaga ttatttgtta      60

actgttaatt gtcct                                                       75


<210>  15
<211>  20
<212>  DNA
<213>  Escherichia coli

<400>  15
gataacggag atcgggaatg                                                  20


<210>  16
<211>  20
<212>  DNA
<213>  Escherichia coli

<400>  16
ctttggctgt cagttcacca                                                  20


<210>  17
<211>  71
<212>  DNA
<213>  Escherichia coli

<400>  17
agcggcaaga ttaaaccagt tcgttcgggc aggtttcgcc tttttccaga tttgtgctat      60

aaacggcgag t                                                           71


<210>  18
<211>  73
<212>  DNA
<213>  Escherichia coli

<400>  18
aggtacttcc atgtcgagta agttagtact ggttctgaac tgcggtagtt tgtgacggaa      60

gatcacttcg cag                                                         73


<210>  19
<211>  75
<212>  DNA
<213>  Escherichia coli

<400>  19
ggtcggcaga acgctgtacc gctttgtagg tggtgttacc ggtgttcaga ttatttgtta      60

actgttaatt gtcct                                                       75


<210>  20
<211>  20
<212>  DNA
<213>  Escherichia coli

<400>  20
cgggacaacg ttcaaaacat                                                  20


<210>  21
<211>  20
<212>  DNA
<213>  Escherichia coli

<400>  21
attgcccatc ttcttgttgg                                                  20


<210>  22
<211>  71
<212>  DNA
<213>  Escherichia coli

<400>  22
ggtcggcaga acgctgtacc gctttgtagg tggtgttacc ggtgttcaga aactaccgca      60

gttcagaacc a                                                           71


<210>  23
<211>  73
<212>  DNA
<213>  Escherichia coli

<400>  23

ccgtgattgg tctgctgacc atcctgaaca tcgtatacaa actgttttaa tgtgacggaa 60

gatcacttcg cag 73

<210> 24
<211> 75
<212> DNA
<213> Escherichia coli

<400> 24
cgcctggggc acgttgcgtt tcgataatct tttcatacaa tcctccggcg ttatttgtta 60

actgttaatt gtcct 75

<210> 25
<211> 21
<212> DNA
<213> Escherichia coli

<400> 25
tgatgagcta cctggtatgg c 21

<210> 26
<211> 24
<212> DNA
<213> Escherichia coli

<400> 26
cgccgacaga gtaataggtt ttac 24

<210> 27
<211> 77
<212> DNA
<213> Escherichia coli

<400> 27
cgcctggggc acgttgcgtt tcgataatct tttcatacaa tcctccggcg ttaaaacagt 60

ttgtatacga tgttcag 77

<210> 28
<211> 72
<212> DNA
<213> Escherichia coli

<400> 28
ataactctaa tgtttaaact cttttagtaa atcacagtga gtgtgagcgc tgtgacggaa 60

gatcacttcg ca 72

<210> 29
<211> 75
<212> DNA
<213> Escherichia coli

<400> 29
ccgtttatgt tgccagacag cgctactgat taagcggatt ttttcgcttt ttatttgtta 60

actgttaatt gtcct                                                    75


<210>  30
<211>  74
<212>  DNA
<213>  Escherichia coli

<400>  30
ccgtttatgt tgccagacag cgctactgat taagcggatt ttttcgcttt gcgctcacac      60

tcactgtgat ttac                                                     74


<210>  31
<211>  21
<212>  DNA
<213>  Escherichia coli

<400>  31
catgctaatg tagccaccaa a                                             21


<210>  32
<211>  20
<212>  DNA
<213>  Escherichia coli

<400>  32
ttgcaccacc atccagataa                                               20


<210>  33
<211>  73
<212>  DNA
<213>  Escherichia coli

<400>  33
aaacgaccac cattaatggt tgtcgaagta cgcagtaaat aaaaaatcca tgtgacggaa      60

gatcacttcg cag                                                      73


<210>  34
<211>  75
<212>  DNA
<213>  Escherichia coli

<400>  34
cggtccgaac ggcgcgccag cacgacgacc gtctggggtg ttacccgttt ttatttgtta      60

actgttaatt gtcct                                                    75


<210>  35
<211>  70
<212>  DNA
<213>  Escherichia coli

<400>  35
aaacgaccac cattaatggt tgtcgaagta cgcagtaaat aaaaaatcca atgcctaagt      60

accgttccgc                                                              70

<210>  36
<211>  74
<212>  DNA
<213>  Escherichia coli

<400>  36
cggtccgaac ggcgcgccag cacgacgacc gtctggggtg ttacccgttt ttaacccccc      60

agtttcgatt tatc                                                         74

<210>  37
<211>  21
<212>  DNA
<213>  Escherichia coli

<400>  37
ctgcggagcc gatctcttta c                                                 21

<210>  38
<211>  22
<212>  DNA
<213>  Escherichia coli

<400>  38
cgagtaataa cgtcctgctg ct                                                22

<210>  39
<211>  72
<212>  DNA
<213>  Escherichia coli

<400>  39
aaacgaccac cattaatggt tgtcgaagta cgcagtaaat aaaaaatcca tgtgacggaa      60

gatcacttcg ca                                                           72

<210>  40
<211>  75
<212>  DNA
<213>  Escherichia coli

<400>  40
cccgccatat tacgaccatg agtggtggtg gcggaacggt acttaggcat ttatttgtta      60

actgttaatt gtcct                                                        75

<210>  41
<211>  70
<212>  DNA
<213>  Escherichia coli

<400>  41
aaacgaccac cattaatggt tgtcgaagta cgcagtaaat aaaaaatcca ttatctctgg      60

cggtgttgac                                                              70

<210>  42
<211>  78
<212>  DNA
<213>  Escherichia coli

<400>  42
cccgccatat tacgaccatg agtggtggtg gcggaacggt acttaggcat tgctgacctc      60

ctggtttaaa cgtacatg                                                    78


<210>  43
<211>  20
<212>  DNA
<213>  Escherichia coli

<400>  43
caaccagatc gagcttgatg                                                  20


<210>  44
<211>  20
<212>  DNA
<213>  Escherichia coli

<400>  44
tgcagaaaac catcgacaag                                                  20


<210>  45
<211>  20
<212>  DNA
<213>  Escherichia coli

<400>  45
caccaatcag cgtgacaact                                                  20


<210>  46
<211>  72
<212>  DNA
<213>  Escherichia coli

<400>  46
gaaggcgaat ggctgagatg aaaaacctga aaattgaggt ggtgcgctat tgtgacggaa      60

gatcacttcg ca                                                          72


<210>  47
<211>  75
<212>  DNA
<213>  Escherichia coli

<400>  47
tctcaggctc cttaccagta cagggcaaca aacaggatta cgatggtggc ttatttgtta      60

actgttaatt gtcct                                                       75


<210>  48

<211>  70
<212>  DNA
<213>  Escherichia coli

<400>  48
gaaggcgaat ggctgagatg aaaaacctga aaattgaggt ggtgcgctat ttatctctgg      60

cggtgttgac                                                              70


<210>  49
<211>  77
<212>  DNA
<213>  Escherichia coli

<400>  49
tctcaggctc cttaccagta cagggcaaca aacaggatta cgatggtggc ttaacggccg      60

ttcaaaatat tttttc                                                       77


<210>  50
<211>  72
<212>  DNA
<213>  Escherichia coli

<400>  50
ctgacgaaac ctcgctccgg cggggttttt tgttatctgc aattcagtac tgtgacggaa      60

gatcacttcg ca                                                           72


<210>  51
<211>  75
<212>  DNA
<213>  Escherichia coli

<400>  51
tctgcgccgg taaagcgctt acgcgtcgat gttgtgcccg aacttgccat ttatttgtta      60

actgttaatt gtcct                                                        75


<210>  52
<211>  70
<212>  DNA
<213>  Escherichia coli

<400>  52
ctgacgaaac ctcgctccgg cggggttttt tgttatctgc aattcagtac ttatctctgg      60

cggtgttgac                                                              70


<210>  53
<211>  70
<212>  DNA
<213>  Escherichia coli

<400>  53
tctgcgccgg taaagcgctt acgcgtcgat gttgtgcccg aacttgccat agctgtttcc      60

tggtttaaac                                                              70

<210>  54
<211>  22
<212>  DNA
<213>  Escherichia coli

<400>  54
gttctgcgcg gaacacgtat ac                                                22


<210>  55
<211>  20
<212>  DNA
<213>  Escherichia coli

<400>  55
ccgctacagg ccatacagac                                                   20


<210>  56
<211>  72
<212>  DNA
<213>  Escherichia coli

<400>  56
tgaactaaga ggaagggaac aacattcaga ccgaaattga atttttttca tgtgacggaa      60

gatcacttcg ca                                                           72


<210>  57
<211>  75
<212>  DNA
<213>  Escherichia coli

<400>  57
ttcacaccct gtgcccgcaa cgcatgtacc acccactgtg cgccattcat ttatttgtta      60

actgttaatt gtcct                                                        75


<210>  58
<211>  70
<212>  DNA
<213>  Escherichia coli

<400>  58
tgaactaaga ggaagggaac aacattcaga ccgaaattga atttttttca ttatctctgg      60

cggtgttgac                                                              70


<210>  59
<211>  71
<212>  DNA
<213>  Escherichia coli

<400>  59
ttcacaccct gtgcccgcaa cgcatgtacc acccactgtg cgccattcat agctgtttcc      60

tggtttaaac g                                                            71

<210>  60
<211>  22
<212>  DNA
<213>  Escherichia coli

<400>  60
gcataagata tcgctgctgt ag                                                22


<210>  61
<211>  20
<212>  DNA
<213>  Escherichia coli

<400>  61
gccagttttg ccagtagcac                                                   20


<210>  62
<211>  72
<212>  DNA
<213>  Escherichia coli

<400>  62
agaacctgat tatgcgccgg atattatcag tcttactcga aaatgaatca tgtgacggaa       60

gatcacttcg ca                                                           72


<210>  63
<211>  75
<212>  DNA
<213>  Escherichia coli

<400>  63
ttcatcgccc acggtctgga tggtcatacg cgataatgtc ggatcgtcgg ttatttgtta       60

actgttaatt gtcct                                                        75


<210>  64
<211>  74
<212>  DNA
<213>  Escherichia coli

<400>  64
agaacctgat tatgcgccgg atattatcag tcttactcga aaatgaatca gacgcgttat       60

tccgcgtgat tggc                                                         74


<210>  65
<211>  21
<212>  DNA
<213>  Escherichia coli

<400>  65
cacaccagag cgagcaacct c                                                 21


<210>  66
<211>  23

<212>  DNA
<213>  Escherichia coli

<400>  66
atgagctgga aagcaaactt agc                                              23


<210>  67
<211>  72
<212>  DNA
<213>  Escherichia coli

<400>  67
acaattatca gtctttatcc ggcgttctaa ggtgtttatc ccactatcac tgtgacggaa      60

gatcacttcg ca                                                          72


<210>  68
<211>  75
<212>  DNA
<213>  Escherichia coli

<400>  68
gcaacacggg tttcattggt taaccgttct cttggtatgc caattcgcat ttatttgtta      60

actgttaatt gtcct                                                       75


<210>  69
<211>  70
<212>  DNA
<213>  Escherichia coli

<400>  69
acaattatca gtctttatcc ggcgttctaa ggtgtttatc ccactatcac ttatctctgg      60

cggtgttgac                                                             70


<210>  70
<211>  71
<212>  DNA
<213>  Escherichia coli

<400>  70
gcaacacggg tttcattggt taaccgttct cttggtatgc caattcgcat agctgtttcc      60

tggtttaaac g                                                           71


<210>  71
<211>  20
<212>  DNA
<213>  Escherichia coli

<400>  71
tcatatcaca ttccttaagc                                                  20


<210>  72
<211>  20
<212>  DNA

<213>  Escherichia coli

<400>  72
atactttgaa cttgttcttt                                                    20


<210>  73
<211>  72
<212>  DNA
<213>  Escherichia coli

<400>  73
cattcatctc gctaacttcg cttattatgg ggatcagttt cagggtttca tgtgacggaa        60

gatcacttcg ca                                                            72


<210>  74
<211>  75
<212>  DNA
<213>  Escherichia coli

<400>  74
gggtgccgtg ggtgtcccac aatgccaata cacttgaaat gattattcat ttatttgtta        60

actgttaatt gtcct                                                         75


<210>  75
<211>  70
<212>  DNA
<213>  Escherichia coli

<400>  75
cattcatctc gctaacttcg cttattatgg ggatcagttt cagggtttca ttatctctgg        60

cggtgttgac                                                               70


<210>  76
<211>  70
<212>  DNA
<213>  Escherichia coli

<400>  76
gggtgccgtg ggtgtcccac aatgccaata cacttgaaat gattattcat agctgtttcc        60

tggtttaaac                                                               70


<210>  77
<211>  20
<212>  DNA
<213>  Escherichia coli

<400>  77
ttcagtacgt cgacgcaggt                                                    20


<210>  78
<211>  20
<212>  DNA
<213>  Escherichia coli

<400>  78
gtaatcgcat ccagagaggg                                                    20


<210>  79
<211>  1095
<212>  DNA
<213>  Lysinibacillus sphaericus

<400>  79
atggaaatct tcaagtatat ggaaaagtat gattatgaac aattggtatt ttgccaagac        60

gaagcatctg ggttaaaagc gattatcgct atccatgaca caacacttgg accagcatta       120

ggtggtgctc gtatgtggac ctacgcgaca gaagaaaatg cgattgagga tgcattaaga       180

ttagcacgcg ggatgacata taaaaatgca gctgctggtt taaaccttgg cggtggaaaa       240

acggtcatta ttggggaccc atttaaagat aaaaacgaag aaatgttccg tgctttaggt       300

cgtttcattc aaggattaaa cggtcgctat attaccgctg aagatgttgg tacaaccgta       360

acagatatgg atttaatcca tgaggaaaca aattacgtta caggtatatc gccagcgttt       420

ggttcatcgg gtaatccttc accagtaact gcttatggcg tttatcgtgg catgaaagca       480

gcggcgaaag aagcatttgg tacggatatg ctagaaggtc gtactatatc ggtacaaggg       540

ctaggaaacg tagcttacaa gctttgcgag tatttacata atgaaggtgc aaaacttgta       600

gtaacagata ttaaccaagc ggctattgat cgtgttgtca atgattttgg cgctacagca       660

gttgcacctg atgaaatcta ttcacaagaa gtcgatattt tctcaccgtg tgcacttggc       720

gcaattttaa atgacgaaac gattccgcaa ttaaaagcaa aagttattgc tggttctgct       780

aataaccaac tacaagattc acgacatgga gattatttac acgagctagg cattgtttat       840

gcacctgact atgtcattaa tgcaggtggt gtaataaatg tcgcggacga attatatggc       900

tataatcgtg aacgagcgtt gaaacgtgta gatggtattt acgatagtat tgaaaaaatc       960

tttgaaattt ccaaacgtga tagtattcca acatatgttg cggcaaatcg tttggcagaa      1020

gaacgtattg ctcgtgtagc gaaatcgcgt agtcagttct taaaaaatga aaaaaatatt      1080

ttgaacggcc gttaa                                                       1095

## Description

### Technical Field

The disclosure relates to a construction method of a recombinant microorganism for producing L-valine, the recombinant microorganism obtained by the construction method, specifically recombinant *Escherichia coli,* and a method for producing L-valine through a fermentation method.

### Background

L-valine, as one of three branched-chain amino acids (BCAA), is an essential amino acid. It cannot be synthesized in humans and animals, and can only be acquired by supplementation in vitro. Nowadays, L-valine has been widely used in the fields of food and medicine, mainly including food additive, nutritional supplement and flavoring agent and the like; and widely used in preparation of cosmetics, as precursors of antibiotics or herbicides and the like. Additionally, along with the increasing demands for feed quality and ratio, the role of L-valine in the feed additive industry will become more and more important in the future. And this will result the expand of its demand and great potential in the future market.

L-valine can be directly synthesized by microbial cells. However, the production capacity of wild-type cells is greatly limited by the intracellular regulatory network, such as feedback inhibition and so on. To obtain a strain capable of efficiently producing L-valine by fermentation process, these self-regulatory mechanisms of the microbial cells must be effectively eliminated. At present, L-valine is mainly produced through fermentation method in the world. Strains used for fermentative production of L-valine are mostly obtained by mutagenesis, and original strains mainly include *Corynebacterium glutamicum, Brevibacterium flavum* and the like. For example, starting from a *Brevibacterium flavum,* Ning Chen et al. produced mutant strains by using protoplast ultraviolet mutagenesis in combination with the DES chemical mutagenesis. Finally, an efficiently L-valine-producing strain TV2564 was screened and selected, and its L-valine titer was as high as 29.39 g/L. But, a notable fact is that strains obtained by traditional mutagenesis have many limits, such as strong randomness, unclear genetic background, producing by-products during fermentation, and not easy to modified for obtaining much more highly efficient strains.

With the rapid development of synthetic biology and metabolic engineering in recent years, recombinant engineered strains have been developed and achieved good results. These strains can efficiently produce L-valine, have clear genetic background, and are easy to be cultivated. Xixian Xie et al. significantly improved L-valine production level of a starting strain VHY03 in the flask fermentation by integrating alsS gene encoding acetolactate synthase of Bacillus substilis, relieving the feedback inhibition of L-valine to key enzymes involved in the synthetic pathway, and integrating the mutant spoTM gene encoding the ppGpp3'-pyrophosphohydrolase of Escherichia coli to enhance the supply of pyruvate. In 2007, Sang Yup Lee's group developed an L-valine-producing strain starting from the Escherichia coli W3110 by in combination of different strategies, such as the rational metabolic engineering, transcriptome analysis and genetic modification, and gene knockout. This strain can produce L-valine in batch culture under aerobic conditions. But it needs continuous oxygen supply during the culture process and L-isoleucine needs to be added to ensure the normal growth. Furthermore, this research group reconstructed a new L-valine producing strain using similar modification strategies from Escherichia coli strain W which has higher tolerance to L-valine. This strain also needs to be cultured under aerobic condition for L-valine production.

Although the titer of L-valine has been improved, the production process of L-valine all need to be carried out under aerobic or microaerobic conditions and unable by a one-step anaerobic fermentation process directly in above reports. The aerobic process requires air during the production process and consumes a lot of energy. More critically, a considerable part of carbon sources enters a tricarboxylic acid cycle (TCA) and is consumed by cell growth resulting that the yield of L-valine is always much lower than the theoretical maximum. Therefore, the aerobic process always has high cost, requirements for devices, and complicated operation steps.

In addition, the overexpression of key genes involved in the metabolic engineering of L-valine strains are always plasmid-borne, so that antibiotics needs to be added in the fermentation process to maintain the existence of the plasmid. As a result, the production cost is greatly increased and a risk of plasmid loss exists in industrial production.

Therefore, there is still a need in the field to provide high-yield, energy-saving, simple and stable recombinant microorganisms for producing L-valine and a corresponding production and preparation method for L-valine.

### Summary

In order to solve problems in the above L-valine fermentation process that reducing power is unbalanced, an aerobic fermentation process is high in cost, and a modified bacterial strain is genetically unstable, the disclosure is capable of, through introducing amino acid dehydrogenase gene, and/or activating activity of a transhydrogenase and/or a NAD kinase, increasing NADPH supply in cell, improving the titer and yield of L-valine produced by *Escherichia coli,* and realizing fermentation-valine production by one-step anaerobic fermentation.

The first aspect of the disclosure is to provide a construction method for construction of a recombinant microorganism producing L-valine. The recombinant microorganism obtained by this method has stable genetic background and balanced L-valine reducing power, and is suitable for one-step anaerobic fermentation.

In the present disclosure, "enhancement" of enzyme activity refers to enhancement of intracellular activity of one or more enzymes which has corresponding gene in the microorganism. The enhancement of the activity may be achieved by any suitable methods known in the field, for example, by overexpression, it includes but not limited to increasing the copy number of the gene or allele, modifying a nucleotide sequence for guiding or controlling gene expression, using a strong promoter, or increasing protein activity or concentration by 10%-500% compared to an initial microbial level.

In one embodiment, an amino acid dehydrogenase gene is transferred into the microorganism, so that the enzyme activity is enhanced.

In one embodiment, the activity of the transhydrogenase in the microorganism is activated, so that the enzyme activity is enhanced.

In one embodiment, the activity of the NAD kinase in the microorganism is activated, so that the enzyme activity is enhanced.

In some preferred embodiments, while the amino acid dehydrogenase gene is transferred into the microorganism, the transhydrogenase in the microorganism is activated; in some preferred embodiments, while the amino acid dehydrogenase gene is transferred into the microorganism, the NAD kinase in the microorganism is activated; and in some more preferred embodiments, while the amino acid dehydrogenase gene is transferred into the microorganism, the transhydrogenase and NAD kinase in the microorganism are activated. In one embodiment, the amino acid dehydrogenase gene transferred in the disclosure is exogenous to the microorganism transferred. The amino acid dehydrogenase gene may be a corresponding gene from any microorganisms such as *Lactococcus,* and *Bacillus.*

In one embodiment, the amino acid dehydrogenase gene is NADH-dependent.

During anaerobic fermentation, glucose metabolism is mainly through the glycolysis pathway (EMP pathway). While 1 mol of glucose is metabolized to generate 2 mol of pyruvate, 2 mol of ATP and 2 mol of NADH may be generated. Therefore, this causes a problem of unbalanced supply of redox power in metabolically engineered *Escherichia coli* under anaerobic conditions, namely, the NADH is excessive, and supply of NADPH is insufficient. Therefore, in order to efficiently produce L-valine under anaerobic conditions, the problem of cofactor imbalance must be solved. The selection of the NADH-dependent amino acid dehydrogenase gene in the disclosure may cause the excessive NADH to be consumed under anaerobic conditions and solve the problem of reducing power imbalance during the anaerobic fermentation.

In one embodiment, the amino acid dehydrogenase gene is a leucine dehydrogenase gene, and preferably, the leucine dehydrogenase gene is leuDH.

In one embodiment, the transhydrogenase is PntAB; and the NAD kinase is YfjB.

In one embodiment, gene encoding the acetohydroxy acid reductoisomerase may also be transferred into the microorganism, so that the enzyme activity is enhanced; and preferably, the acetohydroxy acid reductoisomerase encoding gene is selected from *ilvC.*

The "transfer" may exist in the microorganism in any suitable forms known in the field, for example, in the form of a plasmid or the form of being integrated into a genome. In one embodiment, the enzyme encoding gene integrated into the genome is placed under the control of a suitable regulatory element.

The "activation" may be performed by placing the enzyme encoding gene to be activated under the control of the suitable regulatory element in any modes known in the field, so that the expression of the enzyme gene is enhanced.

The regulatory element is selected from an M1-93 artificial regulatory element, an M1-37 artificial regulatory element or an M1-46 artificial regulatory element.

In one embodiment, the M1-93 artificial regulatory element regulates an *leuDH* gene and a PntAB encoding gene *pntAB.*

In one embodiment, the M1-37 artificial regulatory element regulates an YfjB encoding gene *yfjB.*

In one embodiment, the M1-46 artificial regulatory element regulates an encoding gene *ilvC.*

In one embodiment, the disclosure further includes the following modifications to one or more of the following enzyme genes of the above recombinant microorganism, so that the activity of these enzymes is reduced or inactivated.
(1) Knocking out a methylglyoxal synthase (*mgsA*) gene;
(2) knocking out a lactate dehydrogenase (*IdhA*) gene;
(3) knocking out phosphoacetyl transferase (*pta*) and/or acetate kinase (*ackA*) genes;
(4) knocking out propionate kinase (*tdcD*) and/or formate acetyltransferase (*tdcE*) genes;
(5) knocking out an alcohol dehydrogenase (*adhE*) gene; and
(6) knocking out fumarate reductase (*frd*) and/or pyruvate formate lyase (*pflB*) genes.

It may be understood by those skilled in the art that gene knockout may be performed in a manner known in the prior art, so that the activity of the enzyme is reduced or inactivated. The knockout operation is aimed at an endogenous enzyme gene of the original microorganism, so that the above endogenous enzyme activity of the microorganism is reduced or inactivated.

An encoding sequence of the enzyme gene in the above (1)-(6) may also be substituted with an encoding sequence of another gene by means of genetic engineering such as homologous recombination, thereby the above endogenous enzyme activity of the microorganism is reduced or inactivated. The gene to replace these endogenous enzymes may be a gene to be enhanced for expression, such as the above *ilvC* gene or *leuDH* gene.

In one embodiment, it further includes:
(7) enhancing activity of acetolactate synthase (AHAS) and/or dihydroxy acid dehydratase (*ilvD*) in the recombinant microorganism of the disclosure.

In one preferred embodiment, the AHAS is selected from *ilvBN, ilvGM* or *ilvlH,* and the activity of at least one of them is enhanced.

In one preferred embodiment, the *ilvBN* and *ilvGM* genes may be placed under the control of the appropriate regulatory element, so that the expression of the genes are enhanced. The regulatory element is preferably the M1-93 artificial regulatory element.

In one preferred embodiment, the activity of *ilvlH* is enhanced by releasing the feedback inhibition of valine to the *ilvlH,* for example, the feedback inhibition of valine to the *ilvlH* is released by mutating the *ilvH gene.*

For the involved acetolactate synthase used in biologically synthesizing L-valine, in addition to an isozyme II (herein also referred to as AHAS II), an isozyme III (herein also referred to as AHAS III) is also known. The AHASIII is encoded by an *ilvlH* operon, and the operon is formed by ilvl encoding a large subunit (catalytic subunit) and ilvH encoding a small subunit (control subunit). The AHAS III is feedback inhibited by L-valine. A reported method may be used to mutate the ilvl gene, such as amino acid substitution of ilvH 14 Gly→Asp (Vyazmensky, M. et al., "Biochemistry" 35:10339-10346 (1996)) and/or ilvH 17Ser→Phe (US6737255B2); and ilvH612 (De Felice et al., "Journal of Bacteriology" 120:1058-1067 (1974)) and the like.

In one embodiment, activity of a dihydroxy acid dehydratase (*ilvD*) in the recombinant microorganism of the disclosure is enhanced, for example, the *ilvD* gene is transferred into the microorganism to enhance the activity of the *ilvD.*

An operation in the item (7) is performed optionally in combination with any one or more of the above modifications (1)-(6).

In one embodiment, the operation is performed in combination with the modification of the item (2).

In one embodiment, the operation is performed in combination with the modification of the item (6).

In one embodiment, the operation is performed in combination with the modifications of the item (2) and the item (5).

In one embodiment, the operation is performed in combination with the modifications of the item (2) and the item (6).

In one embodiment, the operation is performed in combination with the modifications of the items (1) and (3)-(6).

In one embodiment, the operation is performed in combination with the modifications of the items (1)-(6).

In one embodiment, optionally, the knockout of the item (1) is achieved by substituting the endogenous *mgsA* gene of the microorganism with the *ilvC* gene.

In one embodiment, the knockout of the item (6) is achieved by substituting the endogenous *pflB* gene of the microorganism with the *ilvD* gene, and/or substituting the endogenous *frd gene* of the microorganism with the *leuDH* gene.

The substitution may be that, in a mode known to those skilled in the art, an encoding sequence of a gene to be inserted is integrated into a substituted gene in the microbial chromosome, so that the gene encoding sequence in the original site is substituted with the encoding sequence of the integrated inserted gene.

Preferably, the substitutions of the ilvC, ilvD and leuDH occur simultaneously.

In one embodiment, the microorganism is *Escherichia coli.*

In one embodiment, the microorganism is *Escherichia coli* ATCC 8739.

In one embodiment, at least one regulatory element is used to regulate the genes encoding the above enzymes involved.

In one embodiment, the regulatory element is selected from an M1-93 artificial regulatory element, an M1-37 artificial regulatory element or an M1-46 artificial regulatory element.

In one embodiment, the M1-93 artificial regulatory element regulates the *ilvD, leuDH, ilvBN, pntAB* and *ilvGM* genes.

In one embodiment, the M1-37 artificial regulatory element regulates the *yfjB* gene.

In one embodiment, the M1-46 artificial regulatory element regulates *ilvC.*

The regulatory element may be inserted at an upstream of the gene by a known genetic engineering method. The method includes, but is not limited to, inserting the sequence of the regulatory element into the upstream of the gene encoding sequence of the target enzyme by means of gene recombination, for example, by means of homologous recombination, so as to enhance the intensity of the target gene expression.

In one embodiment, herein the enzyme encoding gene and the regulatory element are integrated into the genome of the microorganism.

In one embodiment, a plasmid containing the enzyme encoding gene and the regulatory element sequence is transferred into the microorganism.

In one embodiment, the transfer, mutation or knockout of the target enzyme gene is completed by a method of integrating into the genome of the microorganism.

In one embodiment, the transfer, mutation or knockout of the enzyme gene is completed by a method of homologous recombination.

In one embodiment, the transfer, mutation or knockout of the enzyme gene is completed by a method of two-step homologous recombination.

A homologous recombination system known in the field may be used, such as an *Escherichia coli* RecA recombination system and the Red recombination system, is used for the homologous recombination to achieve the transfer, mutation or knockout of the target gene.

The two-step homologous recombination method to transfer, mutate or knock out the target gene includes the following steps (*Escherichia coli are* taken as an example):
(1) Preparation of a DNA fragment I: a pXZ-CS plasmid (Tan, et al., Appl Environ Microbiol, 2013, 79:4838-4844) DNA is used as a template, an amplification primer 1 is used to amplify the DNA fragment I, and used for the first step of homologous recombination;
(2) the first step of the homologous recombination: a pKD46 plasmid (Datsenko and Wanner 2000, Proc Natl Acad Sci USA 97:6640-6645) is transformed into *Escherichia coli,* and then the DNA fragment I is transformed into the *Escherichia coli* containing pKD46, a detection primer 1 is used to verify the transformed bacteria and select the correct colony;
(3) preparation of a DNA fragment II: the original *Escherichia coli* are used as a template, and an amplification primer 2 is used to amplify the DNA fragment II. The DNA fragment II is used for a second step of homologous recombination; and
(4) the second step of the homologous recombination: the DNA fragment II is transformed into the strain obtained in the second step; and a detection primer 2 is used to verify the transformed bacteria and select a correct colony.

The second aspect of the disclosure provides a recombinant microorganism for producing L-valine obtained by using the above construction method, specifically recombinant *Escherichia coli,* it contains an acetohydroxy acid isoreductase and/or an amino acid dehydrogenase gene.

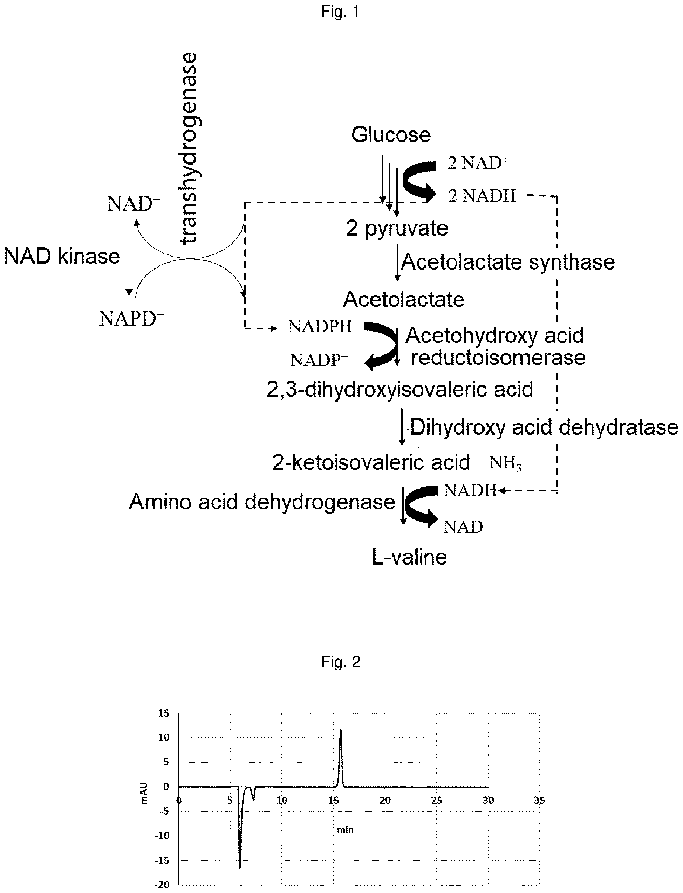

In one embodiment, the *Escherichia coli* ATCC 8739 is used as an original strain, and coupling of intracellular cofactor NADH supply and cell growth is achieved through the gene homologous recombination, thereby coupling (Fig. 1) of the cell growth and L-valine production under anaerobic conditions is achieved.

In one embodiment, the recombinant *Escherichia coli* obtained by the above construction method undergo metabolic evolution, for example, through 50 generations, 70 generations, 80 generations, 90 generations, 100 generations, and 120 generations, the recombinant *Escherichia coli* for highly producing L-valine is obtained. In one embodiment, a recombinant *Escherichia coli* strain producing L-valine is obtained after 70 generations of metabolic evolution. It is preserved in the China General Microbiological Culture Collection Center (CGMCC), and the deposit number is: CGMCC 19456.

The third aspect of the disclosure is an application of the recombinant microorganism obtained by the above method in producing L-valine.

The fourth aspect of the disclosure is a method for fermentatively producing L-valine using the recombinant microorganism obtained by the above construction method, including: (1) fermenting the recombinant microorganism obtained by the above construction method; and (2) separating and harvesting L-valine.

In one embodiment, the fermentation is anaerobic fermentation.

In one embodiment, the anaerobic fermentation includes the following steps:
(1) seed culture: a clone on a plate is picked and inoculated into a seed culture medium at 37°C, and shaked to obtain seed culture solution; and
(2) fermentation culture: the seed culture solution is inoculated into fermentation culture medium, and culturing at 37°C and 150 rpm for 4 days, to obtain fermentation solution. The pH during fermentation process is controlled at 7.0. No air was sparged during the fermentation.

Herein the seed culture medium is formed by the following components (a solvent is water):
Glucose 20 g/L, corn syrup dry powder 10 g/L, KH₂PO₄ 8.8 g/L, (NH₄)₂SO₄ 2.5 g/L, and MgSO₄ •7H₂O 2 g/L.

The fermentation culture medium and the seed culture medium have the same components, and a difference is only that the glucose concentration is 50 g/L.

The beneficial effects of the disclosure:
(1) Compared with previous production methods and strains, the disclosure achieves the one-step anaerobic fermentation production of L-valine, reduces the production cost and improves the transformation rate.
(2) The disclosure preferably constructs a genetically stable L-valine production strain by modified directly on the genome of the recombinant microorganism rather than in the form of plasmid, and additional addition of substances such as antibiotics and inducers does not required, and the production process is stable and easy to operate.
(3) Through metabolic evolution, the titer and yield of L-valine and cell tolerance are improved significantly in the recombinant microorganisms.

### Preservation of Biological Material

Recombinant *Escherichia coli* Sval031 constructed in the disclosure are classified and named: *Escherichia coli.* A biological material is submitted for preservation on March 6, 2020, Depository Authority: China General Microbiological Culture Collection Center (CGMCC), the preservation address is No. 3, Courtyard 1, Beichen West Road, Chaoyang District, Beijing, and the deposit number is CGMCC No. 19456.

### Brief Description of the Drawings

Drawings of the description for constituting a part of the present application are used to provide further understanding of the disclosure. Exemplary embodiments of the disclosure and descriptions thereof are used to explain the disclosure, and do not constitute improper limitation to the disclosure. In the drawings:
Fig. 1: L-valine synthesis pathway.
Fig. 2: Determination of a standard substance of L-valine by high performance liquid chromatography.
Fig. 3: Determination of fermentation solution components of strain Sval030 by high performance liquid chromatography.
Fig. 4: Construction of strain Sval031 by metabolic evolution.
Fig. 5: Determination of a standard substance of L-valine by high performance liquid chromatography.
Fig. 6: Determination of fermentation solution components of strain Sval031 by high performance liquid chromatography.

### Detailed Description of the Embodiments

The disclosure is further described by the following embodiments, but any embodiments or combinations thereof should not be interpreted as limiting a scope or an embodiment of the disclosure. The scope of the disclosure is defined by appended claims. In combination with the description and common knowledge in the field, those of ordinary skill in the art may clearly understand the scope defined by the claims. Without departing from the spirit and scope of the disclosure, those skilled in the art may make any modifications or changes to technical schemes of the disclosure, and such modifications and changes are also included in the scope of the disclosure.

Experimental methods used in the following embodiments are conventional methods unless otherwise specified. Materials, reagents and the like used in the following embodiments may be obtained from commercial sources unless otherwise specified.

Strains and plasmids constructed in this research are shown in Table 1, and primers used are shown in Table 2.

**Table 1: Strains and plasmids used in the disclosure**

| **Strain** | **Related characteristics** | **Sources** |
|---|---|---|
| ATCC 8739 | Wild type | Laboratory preservation |
| M1-93 | ATCC 8739, FRT-Km-FRT::M1-93::lacZ | Lu, et al., Appl Microbiol Biotechnol, 2012,93:2455-2462 |
| M1-46 | ATCC 8739, FRT-Km-FRT::M1-46::IacZ | Lu, et al., Appl Microbiol Biotechnol, 2012,93:2455-2462 |
| M1-37 | ATCC 8739, FRT-Km-FRT::M1-37::IacZ | Lu, et al., Appl Microbiol Biotechnol,2012,93:2455-2462 |
| Sval001 | ATCC 8739, mgsA::cat-sacB | Constructed by the disclosure |
| Sval002 | Sval001, ΔmgsA | Constructed by the disclosure |
| Sval003 | Sval002, IdhA::cat-sacB | Constructed by the disclosure |
| Sval004 | Sval003, ΔIdhA | Constructed by the disclosure |
| Sval005 | Sval004, ackA-pta::cat-sacB | Constructed by the disclosure |
| Sval006 | Sval005, Δ ackA-pta | Constructed by the disclosure |
| Sval007 | Sval006, tdcDE::cat-sacB | Constructed by the disclosure |
| Sval008 | Sval007, ΔtdcDE | Constructed by the disclosure |
| Sval009 | Sval008, adhE::cat-sacB | Constructed by the disclosure |
| Sval010 | Sval009, ΔadhE | Constructed by the disclosure |
| Sval011 | Sval010, mgsA::cat-sacB | Constructed by the disclosure |
| Sval012 | Sval011, mgsA::ilvC | Constructed by the disclosure |
| Sval013 | Sval012, mgsA::cat-sacB::ilvC | Constructed by the disclosure |
| Sval014 | Sval013, mgsA::M1-46-ilvC | Constructed by the disclosure |
| Sval015 | Sval014, pflB::cat-sacB | Constructed by the disclosure |
| Sval016 | Sval015, pfIB::ilvD | Constructed by the disclosure |
| Sval017 | Sval016, pfIB::cat-sacB::ilvD | Constructed by the disclosure |
| Sval018 | Sval017, pfIB::RBS4-ilvD | Constructed by the disclosure |
| Sval019 | Sval018, cat-sacB::ilvB | Constructed by the disclosure |
| Sval020 | Sval019, M1-93:: ilvB | Constructed by the disclosure |
| Sval021 | Sval020, cat-sacB::ilvG | Constructed by the disclosure |
| Sval022 | Sval021, M1-93:: ilvG | Constructed by the disclosure |
| Sval023 | Sval022, ilvH::cat-sacB | Constructed by the disclosure |
| Sval024 | Sval023, ilvH:: ilvH* | Constructed by the disclosure |
| Sval025 | Sval024, frd::cat-sacB | Constructed by the disclosure |
| Sval026 | Sval025, frd::M1-93-leuDH | Constructed by the disclosure |
| Sval027 | Sval026, cat-sacB::pntAB | Constructed by the disclosure |
| Sval028 | Sval027, M1-93-pntAB | Constructed by the disclosure |
| Sval029 | Sval028, cat-sacB::yfjB | Constructed by the disclosure |
| Sval030 | Sval029, M1-37-yfjB | Constructed by the disclosure |
| Sval031 | metabolic evolution of Sval030 for 70 generations, CGMCC 19456 | Constructed by the disclosure |

| **Plasmid** | **Related characteristics** | **Sources** |
|---|---|---|
| pUC57-M1-93-leuDH | Artificial regulatory element M1-93 and chemically synthesized gene leuDH are linked to a pUC57 vector together | Nanjing Genscript Biotechnology Co., Ltd. |

**Table 2: Primers used in the disclosure**

| **Primer name** | **Sequence** | **Seque nce numb er** |
|---|---|---|
| mgsA-cs-up | gtaggaaagttaactacggatgtacattatggaactgacgactcgcacttTG TGACGGAAGATCACTTCGCAG | 1 |
| mgsA-cs-do wn | gcgtttgccacctgtgcaatattacttcagacggtccgcgagataacgctTT ATTTGTTAACTGTTAATTGTCCT | 2 |
| XZ-mgsA-u p | cag ctcatcaaccag gtcaa | 3 |
| XZ-mgsA-d own | aaaagccgtcacgttattgg | 4 |
| mgsA-del-d own | gcgtttgccacctgtgcaatattacttcagacggtccgcgagataacgctaag tgcgagtcgtcagttcc | 5 |
| mgsA-ilvC-u p | gtaggaaagttaactacggatgtacattatggaactgacgactcgcacttAT GGCTAACTACTTCAATACac | 6 |
| mgsA-ilvC-d own | gcgtttgccacctgtgcaatattacttcagacggtccgcgagataacgctTT AACCCGCAACAGCAATACGtttc | 7 |
| mgsA-Pcs-u p | gtaggaaagttaactacggatgtacattatggaactgacgactcgcacttTG TGACGGAAGATCACTTCGCAG | 8 |
| mgsA-Pcs-d own | agctgtgccagctgctggcgcagattcagtGTATTGAAGTAGTTAG CCATTTATTTGTTAACTGTTAATTGTCCT | 9 |
| mgsA-P46-u p | gtaggaaagttaactacggatgtacattatggaactgacgactcgcacttTT ATCTCTGGCGGTGTTGAC | 10 |
| ilvC-P46-do wn | agctgtgccagctgctggcgcagattcagtGTATTGAAGTAGTTAG CCATAGCTGTTTCCTGGTTTAAACCG | 11 |
| ilvC-YZ347-down | cgcactacatcagagtgctg | 12 |
| IdhA-cs-up | ttcaacatcactggagaaagtcttatgaaactcgccgtttatagcacaaaTG TGACGGAAGATCACTTCGCAG | 13 |
| IdhA-cs-dow n | agcggcaagattaaaccagttcgttcgggcaggtttcgcctttttccagaTTA TTTGTTAACTGTTAATTGTCCT | 14 |
| XZ-IdhA-up | GATAACGGAGATCGGGAATG | 15 |
| XZ-IdhA-down | CTTTGGCTGTCAGTTCACCA | 16 |
| IdhA-del-do wn | agcggcaagattaaaccagttcgttcgggcaggtttcgcctttttccagatttgt gctataaacggcgagt | 17 |
| ackA-cs-up | aggtacttccatgtcgagtaagttagtactggttctgaactgcggtagttTGT GACGGAAGATCACTTCGCAG | 18 |
| pta-cs-down | ggtcggcagaacgctgtaccgctttgtaggtggtgttaccggtgttcagaTT ATTTGTTAACTGTTAATTGTCCT | 19 |
| XZ-ackA-up | cgggacaacgttcaaaacat | 20 |
| XZ-pta-dow n | attgcccatcttcttgttgg | 21 |
| ackA-del-do wn | ggtcggcagaacgctgtaccgctttgtaggtggtgttaccggtgttcagaaac taccgcagttcagaacca | 22 |
| tdcDE-cs-up | ccgtgattggtctgctgaccatcctgaacatcgtatacaaactgttttaaTGT GACGGAAGATCACTTCGCAG | 23 |
| tdcDE-cs-do wn | cgcctggggcacgttgcgtttcgataatctttttcatacatcctccggcgTTAT TTGTTAACTGTTAATTGTCCT | 24 |
| XZ-tdcDE-u P | TGATGAGCTACCTGGTATGGC | 25 |
| XZ-tdcDE-d own | CGCCGACAGAGTAATAGGTTTTAC | 26 |
| tdcDE-del-d own | cgcctggggcacgttgcgtttcgataatctttttcatacatcctccggcgttaaa acagtttgtatacgatgttcag | 27 |
| adhE-cs-up | ATAACTCTAATGTTTAAACTCTTTTAGTAAATCACAGT GAGTGTGAGCGCTGTGACGGAAGATCACTTCGCA | 28 |
| adhE-cs-do wn | CCGTTTATGTTGCCAGACAGCGCTACTGATTAAGCG GATTTTTTCGCTTTTTATTTGTTAACTGTTAATTGTCC T | 29 |
| adhE-del-do wn | CCGTTTATGTTGCCAGACAGCGCTACTGATTAAGCG GATTTTTTCGCTTTGCGCTCACACTCACTGTGATTTA C | 30 |
| XZ-adhE-up | CATGCTAATGTAGCCACCAAA | 31 |
| XZ-adhE-do wn | TTGCACCACCATCCAGATAA | 32 |
| pfIB-CS-up | aaacgaccaccattaatggttgtcgaagtacgcagtaaataaaaaatccaT GTGACGGAAGATCACTTCGCAG | 33 |
| pflB-CS-do wn | CGGTCCGAACGGCGCGCCAGCACGACGACCGTCT GGGGTGTTACCCGTTTTTATTTGTTAACTGTTAATTGT CCT | 34 |
| pflB-ilvD-up | aaacgaccaccattaatggttgtcgaagtacgcagtaaataaaaaatccaa tgcctaagtaccgttccgc | **35** |
| pflB-ilvD-do wn | CGGTCCGAACGGCGCGCCAGCACGACGACCGTCT GGGGTGTTACCCGTTTttaaccccccagtttcgatttatc | **36** |
| XZ-pflB-up6 00 | CTGCGGAGCCGATCTCTTTAC | 37 |
| XZ-pflB-dow n | CGAGTAATAACGTCCTGCTGCT | 38 |
| pflB-Pcs-up | aaacgaccaccattaatggttgtcgaagtacgcagtaaataaaaaatccaT GTGACGGAAGATCACTTCGCA | 39 |
| pflB-Pcs-do wn | CCCGCCATATTACGACCATGAGTGGTGGTGGCGGAA CGGTACTTAGGCATTTATTTGTTAACTGTTAATTGTCC T | 40 |
| pflB-Pro-up | AAACGACCACCATTAATGGTTGTCGAAGTACGCAGT AAATAAAAAATCCATTATCTCTGGCGGTGTTGAC | 41 |
| ilvD-Pro-do wn | cccgccatattacgaccatgagtggtggtggcggaacggtacttaggcatT GCTGACCTCCTGGTTTAAACGTACATG | 42 |
| ilvD-YZ496-down | caaccagatcgagcttgatg | 43 |
| XZ-frd-up | TGCAGAAAACCATCGACAAG | 44 |
| XZ-frd-down | CACCAATCAGCGTGACAACT | 45 |
| frd-cs-up | GAAGGCGAATGGCTGAGATGAAAAACCTGAAAATTG AGGTGGTGCGCTATTGTGACGGAAGATCACTTCGCA | 46 |
| frd-cs-down | TCTCAGGCTCCTTACCAGTACAGGGCAACAAACAGG ATTACGATGGTGGCTTATTTGTTAACTGTTAATTGTCC | 47 |
| | T | |
| frd-M93-up | GAAGGCGAATGGCTGAGATGAAAAACCTGAAAATTG AGGTGGTGCGCTATTTATCTCTGGCGGTGTTGAC | 48 |
| frd-leuDH-d own | TCTCAGGCTCCTTACCAGTACAGGGCAACAAACAGG ATTACGATGGTGGCTTAACGGCCGTTCAAAATATTTT TTTC | 49 |
| ilvB pro-catup | ctgacgaaacctcgctccggcggggtttttgttatctgcaattcagtacTGT GACGGAAGATCACTTCGCA | 50 |
| ilvB pro-catdown | tctgcgccggtaaagcgcttacgcgtcgatgttgtgcccgaacttgccatTTA TTTGTTAACTGTTAATTGTCCT | 51 |
| ilvB pro-up | ctgacgaaacctcgctccggcggggtttttgttatctgcaattcagtacTTAT CTCTGGCGGTGTTGAC | 52 |
| ilvB pro-down | tctgcgccggtaaagcgcttacgcgtcgatgttgtgcccgaacttgccatAG CTGTTTCCTGGTTTAAAC | 53 |
| ilvB pro-YZup | gttctgcgcggaacacgtatac | 54 |
| ilvB pro-YZdown | ccgctacaggccatacagac | 55 |
| ilvG pro-catup | tgaactaagaggaagggaacaacattcagaccgaaattgaatttttttcaT GTGACGGAAGATCACTTCGCA | 56 |
| ilvG pro-catdown | ttcacaccctgtgcccgcaacgcatgtaccacccactgtgcgccattcatTT ATTTGTTAACTGTTAATTGTCCT | 57 |
| ilvG pro-up | tgaactaagaggaagggaacaacattcagaccgaaattgaatttttttcaTT ATCTCTGGCGGTGTTGAC | 58 |
| ilvG pro-down | ttcacaccctgtgcccgcaacgcatgtaccacccactgtgcgccattcatAG CTGTTTCCTGGTTTAAACG | 59 |
| ilvG pro-YZup | gcataagatatcgctgctgtag | 60 |
| ilvG p-YZdown | gccagttttgccagtagcac | 61 |
| ilvH*-cat-up | agaacctgattatgCGCCGGATATTATCAGTCTTACTCGAA AATGAATCATGTGACGGAAGATCACTTCGCA | 62 |
| ilvH*-cat-do wn | TTCATCGCCCACGGTCTGGATGGTCATACGCGATAA TGTCGGATCGTCGGTTATTTGTTAACTGTTAATTGTC CT | 63 |
| ilvH*-mut-up | agaacctgattatgCGCCGGATATTATCAGTCTTACTCGAA AATGAATCAGaCGCGTTATtCCGCGTGATTGGC | 64 |
| ilvH*-mut-do wn | CACACCAGAGCGAGCAACCTC | 65 |
| ilvH*-mutYZ -up | atg ag ctg g aaag caaacttag c | 66 |
| pntAB-cs-up | acaattatcagtctttatccggcgttctaaggtgtttatcccactatcacTGTG ACGGAAGATCACTTCGCA | 67 |
| pntAB-cs-do wn | gcaacacgggtttcattggttaaccgttctcttggtatgccaattcgcatTTAT TTGTTAACTGTTAATTGTCCT | 68 |
| pntAB-P-up | acaattatcagtctttatccggcgttctaaggtgtttatcccactatcacTTAT CTCTGGCGGTGTTGAC | 69 |
| pntAB-M93-down | gcaacacgggtttcattggttaaccgttctcttggtatgccaattcgcatAGC TGTTTCCTGGTTTAAACG | 70 |
| pntAB-YZ-u p | tcatatcacattccttaagc | 71 |
| pntAB-YZ-d own | atactttgaacttgttcttt | 72 |
| yfjb-cs-up | cattcatctcgctaacttcgcttattatggggatcagtttcagggtttcaTGTG ACGGAAGATCACTTCGCA | 73 |
| yfjb-cs-dow | gggtgccgtgggtgtcccacaatgccaatacacttgaaatgattattcatTTA | 74 |
| n | TTTGTTAACTGTTAATTGTCCT | |
| yfjb-P-up | cattcatctcgctaacttcgcttattatggggatcagtttcagggtttcaTTATC TCTGGCGGTGTTGAC | 75 |
| yfjb-M37-do wn | gggtgccgtgggtgtcccacaatgccaatacacttgaaatgattattcatAG CTGTTTCCTGGTTTAAAC | 76 |
| yfjb-YZ-up | ttcagtacgtcgacgcaggt | 77 |
| yfjb-YZ-dow n | gtaatcgcatccagagaggg | 78 |

### Example 1: Knockout of methylglyoxal synthase encoding gene mgsA in ATCC 8739 strain

Started from *Escherichia coli* ATCC 8739, a two-step homologous recombination method is used to knock out the methylglyoxal synthase encoding gene *mgsA,* and specific steps are as follows.

In a first step, a pXZ-CS plasmid DNA is used as a template, 2719 bp of a DNA fragment I is amplified by using primers mgsA-cs-up/mgsA-cs-down, and used for the first step of homologous recombination.

An amplification system is: Phusion 5X buffer (NewEngland Biolabs) 10 µl, dNTP (10 mM for each dNTP) 1 µl, DNA template 20 ng, primers (10 µM) 2 µl each, Phusion High-Fidelity DNA polymerase (2.5 U/µl) 0.5 µl, distilled water 33.5 µl, and a total volume is 50 µl.

Amplification conditions are 98°C pre-denaturation for 2 minutes (1 cycle); 98°C denaturation for 10 seconds, 56°C annealing for 10 seconds, 72°C extension for 2 minutes (30 cycles); and 72°C extension for 10 minutes (1 cycle).

The above DNA fragment I is used for the first homologous recombination: firstly, a pKD46 plasmid (purchased from the Coil Genetic Stock Center (CGSC) of Yale University, CGSC#7739) is transformed into *Escherichia coli* ATCC 8739 by an electrotransformation method, and then the DNA fragment I is electrotransformed to the *Escherichia coli* ATCC 8739 with the pKD46.

Electrotransformation conditions are as follows: firstly, electrotransformation competent cells of the *Escherichia coli ATCC* 8739 with the pKD46 plasmid are prepared; 50 µl of the competent cells are placed on ice, and 50 ng of the DNA fragment I is added. The mixture was placed on ice for 2 minutes, and transferred into a 0.2 cm MicroPulser Electroporation Cuvette (Bio-Rad). The electroporation was carried with the MicroPulser (Bio-Rad) electroporation apparatus and the electric voltage was 2.5 kV. After electric shock, 1 ml of LB medium was quickly added into the electroporation cuvette, and transferred into a test tube after pipetting five times. The culture was incubated at 30°C with shaking at 75 rpm for 2 hours. 200 µl of culture was spread onto a LB plate containing ampicillin (a final concentration is 100 µg/ml) and chloramphenicol (a final concentration is 34 µg/ml). After being cultured overnight at 30°C, colonies were verified with primer set XZ-mgsA-up/XZ-mgsA-down, and a correct colony amplification product is a 3646 bp fragment. A correct single colony was selected, and named as Sval001.

In a second step, a genomic DNA of wild-type *Escherichia coli* ATCC 8739 is used as template, and 566 bp of a DNA fragment II is amplified with primer set XZ-mgsA-up/mgsA-del-down. DNA fragment II is used for the second homologous recombination. Amplification conditions and system are the same as those described in the first step. The DNA fragment II is electrotransformed into the strain Sval001.

Electrotransformation conditions are as follows: firstly, electrotransformation competent cells of the Sval001 with the pKD46 plasmid (Dower et al., 1988, Nucleic Acids Res 16:6127-6145) were prepared; 50 µl of the competent cells were placed on ice, and 50 ng of a DNA fragment II is added. The mixture was placed on ice for 2 minutes, and transferred into a 0.2 MicroPulser Electroporation Cuvette (Bio-Rad). The electroporation was carried with the MicroPulser (Bio-Rad) electroporation apparatus and the electric voltage was 2.5 kV. After electric shock, 1 ml of LB medium was quickly added into the electroporation cuvette, and transferred into a test tube after pipetting five times. The culture was incubated at 30°C with shaking at 75 rpm for 4 hours. The culture was then transferred into LB medium containing 10% sucrose but without a sodium chloride (50 ml of a medium is loaded in 250 ml of a flask), and after being cultured for 24 hours, it is streak-cultured on an LB solid medium containing 6% sucrose without a sodium chloride. The correct clone was verified by colony PCR amplification with primer set XZ-mgsA-up/XZ-mgsA-down, and a correct colony amplification product was 1027 bp. A correct single colony is then selected, and named as Sval002 (Table 1).

### Example 2: Knockout of lactate dehydrogenase encoding gene IdhA

Started from Sval002, and a lactate dehydrogenase encoding gene *IdhA* is knocked out by a two-step homologous recombination method. Specific steps are as follows.

In a first step, a pXZ-CS plasmid DNA is used as a template, 2719 bp of a DNA fragment I is amplified by using primers IdhA-cs-up/IdhA-cs-down, and used for the first step of the homologous recombination. Amplification system and amplification conditions are the same as those described in Example 1. The DNA fragment I is electrotransformed to the Sval002.

The DNA fragment I is used for the first homologous recombination: firstly, a pKD46 plasmid is transformed into *Escherichia coli* Sval002 by an electrotransformation method, and then the DNA fragment I is electrotransformed into the *Escherichia coli* Sval002 with the pKD46.

Electrotransformation conditions and steps are the same as the first step method for the *mgsA* gene knockout described in example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primer set XZ-IdhA-up/XZ-IdhA-down, and a correct PCR product should be 3448 bp. A correct single colony is picked, and named as Sval003.

In a second step, a DNA of wild-type *Escherichia coli* ATCC 8739 is used as a template, and 476 bp of a DNA fragment II is amplified with primers XZ-IdhA-up/IdhA-del-down. The DNA fragment II is used for the second homologous recombination. The DNA fragment II is electrotransformed into the strain Sval003.

Electrotransformation conditions and steps are the same as the second step method for the *mgsA* gene knockout described in example 1. Colonies were verified by PCR using primers XZ-IdhA-up/XZ-IdhA-down and sequenced, and a correct colony amplification product is 829 bp. A correct single colony is picked, and named as Sval004 (Table 1).

### Example 3: Knockout of phosphoacetyl transferase encoding gene pta and acetate kinase encoding gene ackA

Started from Sval004, a two-step homologous recombination method is used to knock out a phosphoacetyl transferase encoding gene *pta* and an acetate kinase encoding gene *ackA.* Specific steps are as follows.

In a first step, a pXZ-CS plasmid DNA is used as a template, 2719 bp of a DNA fragment I is amplified by using primers ackA-cs-up/pta-cs-down and used for the first step of the homologous recombination. Amplification system and amplification conditions are the same as those described in example 1. The DNA fragment I is electrotransformed to the Sval004. The DNA fragment I is used for the first homologous recombination: firstly, a pKD46 plasmid is transformed into *Escherichia coli* Sval004 by an electrotransformation method, and then the DNA fragment I is electrotransformed into the *Escherichia coli* Sval004 with the pKD46.

Electrotransformation conditions and steps are the same as the first step method for the *mgsA* gene knockout described in example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers XZ-ackA-up/XZ-pta-down, and a correct PCR product should be 3351 bp. A correct single colony is picked, and named as Sval005.

In a second step, a DNA of wild-type *Escherichia coli* ATCC 8739 is used as a template, and 371 bp of a DNA fragment II is amplified with primers XZ-ackA-up/ackA-del-down. The DNA fragment II is used for the second homologous recombination. The DNA fragment II is electrotransformed into the strain Sval005.

Electrotransformation conditions and steps are the same as the second step method for the *mgsA* gene knockout described in example 1. Colony PCR is used to verify clones using primers XZ-ackA-up/XZ-pta-down, and a correct colony amplification product is 732 bp. A correct single colony is picked, and named as Sval006 (Table 1).

### Example 4: Knockout of propionate kinase encoding gene tdcD and formate acetyltransferase encoding gene tdcE

Started from Sval006, a two-step homologous recombination method is used to knock out the propionate kinase encoding gene *tdcD* and the formate acetyltransferase encoding gene *tdcE.* Specific steps are as follows.

In a first step, a pXZ-CS plasmid DNA is used as a template, 2719 bp of a DNA fragment I is amplified by using primers tdcDE-cs-up/tdcDE-cs-down, and used for the first step of the homologous recombination. Amplification system and amplification conditions are the same as those described in example 1. The DNA fragment I is electrotransformed to the Sval006.

The DNA fragment I is used for the first homologous recombination: firstly, a pKD46 plasmid is transformed into *Escherichia coli* Sval006 by an electrotransformation method, and then the DNA fragment I is electrotransformed into the *Escherichia coli* Sval006 with the pKD46.

Electrotransformation conditions and steps are the same as the first step method for the *mgsA* gene knockout described in example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-tdcDE-up/ XZ-tdcDE-down and a correct PCR product should be 4380 bp. A correct single colony is picked, and named as Sval007.

In a second step, a DNA of wild-type *Escherichia coli* ATCC 8739 is used as a template, and 895 bp of a DNA fragment II is amplified with primers XZ-tdcDE-up/tdcDE-del-down. The DNA fragment II is used for the second homologous recombination. The DNA fragment II is electrotransformed into strain Sval007.

Electrotransformation conditions and steps are the same as the second step method for the *mgsA* gene knockout described in example 1. Colonies were PCR verified using primers XZ-tdcDE-up/ XZ-tdcDE-down, and a correct colony amplification product is 1761 bp. A correct single colony is picked, and named as Sval008 (Table 1).

### Example 5: Knockout of alcohol dehydrogenase gene adhE

Started from Sval008, a two-step homologous recombination method is used to knock out the alcohol dehydrogenase gene *adhE.* Specific steps are as follows.

In a first step, a pXZ-CS plasmid DNA is used as a template, 2719 bp of a DNA fragment I is amplified by using primers adhE-cs-up /adhE-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval008 by an electrotransformation method, and then the DNA fragment I is electrotransformed into the *Escherichia coli* Sval008 with the pKD46.

Electrotransformation conditions and steps are the same as the first step method for the *mgsA* gene knockout described in example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-adhE-up/XZ-adhE-down, and a correct PCR product should be 3167 bp. A correct single colony is picked, and named as Sval009.

In a second step, a DNA of wild-type *Escherichia coli* ATCC 8739 is used as a template, and 271 bp of a DNA fragment II is amplified with primers XZ-adhE-up/XZ-adhE-down. The DNA fragment II is used for the second homologous recombination. The DNA fragment II is electrotransformed into the strain Sval009.

Electrotransformation conditions and steps are the same as the second step method for the *mgsA* gene knockout described in Example 1. Colonies were PCR verified using primers XZ-adhE-up/XZ-adhE-down, and a correct colony amplification product is 548 bp. A correct single colony is picked, and named as Sval010 (Table 1).

### Example 6: Integration of acetohydroxy acid reductoisomerase encoding gene ilvC in methylglyoxal synthase encoding gene mgsA site

Started from Sval010, an acetohydroxy acid reductoisomerase encoding gene *ilvC* from *Escherichia coli* is integrated into the methylglyoxal synthase encoding gene *mgsA* site through a two-step homologous recombination method. Specific steps are as follows.

In a first step, a *cat-sacB* fragment is integrated into the *mgsA* site of strain Sval010. PCR, integration, and verification of the *cat-sacB* fragment are exactly the same as the first step of the *mgsA* gene knockout in example 1, and an obtained clone is named as Sval011.

In a second step, a DNA of wild-type *Escherichia coli* ATCC 8739 is used as a template, 1576 bp of a DNA fragment II is amplified by using primers mgsA-ilvC-up/mgsA-ilvC-down. The DNA fragment II is used for the second homologous recombination. The DNA fragment II is electrotransformed into the strain Sval011.

Electrotransformation conditions and steps are the same as the second step method for the *mgsA* gene knockout described in example 1. Colonies were PCR verified using primers XZ-mgsA-up/XZ-mgsA-down and sequenced, and a correct colony amplification product is 2503 bp. A correct single colony is picked, and named as Sval012 (Table 1).

### Example 7: Regulation of acetohydroxy acid reductoisomerase encoding gene ilvC

Started from Sval012, and an artificial regulatory element is used to regulate expression of the acetohydroxy acid reductoisomerase encoding gene *ilvC* integrated in methylglyoxal synthase encoding gene *mgsA* site. Specific steps are as follows.

In a first step, a pXZ-CS plasmid DNA is used as a template, 2719 bp of a DNA fragment I is amplified by using primers mgsA-Pcs-up/mgsA-Pcs-down, and used for the first step of the homologous recombination. Amplification system and amplification conditions are the same as those described in example 1. The DNA fragment I is electrotransformed into the Sval012.

The DNA fragment I is used for the first homologous recombination: firstly, a pKD46 plasmid (purchased from the Coil Genetic Stock Center (CGSC) of Yale University, CGSC#7739) is transformed into *Escherichia coli* SvaI012 by an electrotransformation method, and then the DNA fragment I is electrotransformed into the *Escherichia coli* Sval012 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were selected for PCR verification, primers XZ-mgsA-up/ilvC-YZ347-down are used for the verification, and a correct PCR product should be 3482 bp. A correct single colony is picked, and named as Sval013.

In a second step, a genomic DNA of M1-46 (Lu, et al., Appl Microbiol Biotechnol, 2012,93:2455-2462) is used as a template, and 188 bp of a DNA fragment II is amplified by using primers mgsA-P46-up/ilvC-P46-down. The DNA fragment II is used for the second homologous recombination. The DNA fragment II is electrotransformed into the strain Sval013.

Electrotransformation conditions and steps are the same as the second step method for the *mgsA* gene knockout described in Example1. Colonies were PCR verified using primers XZ-mgsA-up/ilvC-YZ347-down and sequenced, and a correct colony amplification product is 951 bp. A correct single colony is picked, and named as Sval014 (Table 1).

### Example 8: Integration of dihydroxy acid dehydratase encoding gene ilvD

Started from Sval014, a dihydroxy acid dehydratase encoding gene *ilvD from Escherichia coli* is integrated into the pyruvate formate lyase encoding gene *pflB* site and replaces the *pflB* gene through a two-step homologous recombination method, namely the *pflB* gene is knocked out while the *ilvD* is integrated. Specific steps are as follows.

In a first step, a pXZ-CS plasmid DNA is used as a template, 2719 bp of a DNA fragment I is amplified by using primers pflB-CS-up/pflB-CS-down, and used for the first step of the homologous recombination. Amplification system and amplification conditions are the same as those described in example 1. The DNA fragment I is electrotransformed into the Sval014.

The DNA fragment I is used for the first homologous recombination: firstly, a pKD46 plasmid is transformed into *Escherichia coli* Sval014 by an electrotransformation method, and then the DNA fragment I is electrotransformed into the *Escherichia coli* Sval014 with the pKD46.

Electrotransformation conditions and steps are the same as the first step method for the *mgsA* gene knockout described in Example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers XZ-pflB-up600/XZ-pflB-down, and a correct PCR product should be 3675 bp. A correct single colony is picked, and named as Sval015.

In a second step, a genomic DNA of *Escherichia coli* MG1655 (from ATCC, No. 700926) is used as a template, and 1951 bp of a DNA fragment II is amplified by using primers pflB-ilvD-up/pflB-ilvD-down. The DNA fragment II is used for the second homologous recombination. The DNA fragment II is electrotransformed into the strain Sval015.

Electrotransformation conditions and steps are the same as the second step method for the *mgsA* gene knockout described in Example 1. Colonies were PCR verified using primers XZ-pflB-up600/XZ-pflB-down and sequenced, and a correct colony amplification product is 2907 bp. A correct single colony is picked, and named as Sval016 (Table 1).

### Example 9: Regulation of dihydroxy acid dehydratase encoding gene ilvD

Started from Sval016, and an artificial regulatory element is used to regulate expression of the dihydroxy acid dehydratase encoding gene *ilvD* integrated in the pyruvate formate lyase encoding gene *pflB* site. Specific steps are as follows.

In a first step, a pXZ-CS plasmid DNA is used as a template, 2719 bp of a DNA fragment I is amplified by using primers pflB-Pcs-up/pflB-Pcs-down, and used for the first step of the homologous recombination. Amplification system and amplification conditions are the same as those described in Example 1. The DNA fragment I is electrotransformed into the Sval016.

The DNA fragment I is used for the first homologous recombination: firstly, a pKD46 plasmid is transformed into *Escherichia coli* Sval016 by an electrotransformation method, and then the DNA fragment I is electrotransformed into the *Escherichia coli* Sval016 with the pKD46.

Electrotransformation conditions and steps are the same as the first step method for the *mgsA* gene knockout described in Example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers XZ-pflB-up600/ilvD-YZ496-down, and a correct PCR product should be 3756 bp. A correct single colony is picked, and named as Sval017.

In a second step, a genomic DNA of M1-93 (Lu, et al., Appl Microbiol Biotechnol, 2012,93:2455-2462) is used as a template, and 189 bp of a DNA fragment II is amplified by using primers pflB-Pro-up/ilvD-Pro-down. The DNA fragment II is used for the second homologous recombination. The DNA fragment II is electrotransformed into the strain Sval017.

Electrotransformation conditions and steps are the same as the second step method for the *mgsA* gene knockout described in Example 1. Colonies were PCR verified using primers XZ-pflB-up600/ilvD-YZ496-down and sequenced, and a correct colony amplification product is 1226 bp. A correct single colony is picked, and named as Sval018 (Table 1).

### Example 10: Regulation of acetolactate synthase gene ilvBN

An artificial regulatory element M1-93 is used to regulate expression of an acetolactate synthase gene *ilvBN* through a two-step homologous recombination method. Specific steps are as follows.

In a first step, a pXZ-CS plasmid DNA is used as a template, 2719 bp of a DNA fragment I is amplified by using primers ilvB pro-catup/ilvB pro-catdown, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval018 by an electrotransformation method, and then the DNA fragment I is electrotransformed into the *Escherichia coli* Sval018 with the pKD46.

Electrotransformation conditions and steps are the same as the first step method for the *mgsA* gene knockout described in Example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers ilvB pro-YZup/ilvB pro-YZdown, and a correct PCR product should be 2996 bp. A correct single colony is picked, and named as Sval019.

In a second step, a genomic DNA of M1-93 is used as a template, and 188 bp of a DNA fragment II is amplified by using primers ilvB pro-up/ilvB pro-down. The DNA fragment II is used for the second homologous recombination. The DNA fragment II is electrotransformed into the strain Sval019.

Electrotransformation conditions and steps are the same as the second step method for the *mgsA* gene knockout described in Example 1. Colonies were PCR verified using primers ilvB pro-YZup/ilvB pro-YZdown and sequenced, and a correct colony amplification product is 465 bp. A correct single colony is picked, and named as Sval020.

### Example 11: Regulation of acetolactate synthase gene ilvGM

An artificial regulatory element M1-93 is used to regulate expression of the acetolactate synthase gene *ilvGM* through a two-step homologous recombination method. Specific steps are as follows.

In a first step, a pXZ-CS plasmid DNA is used as a template, 2719 bp of a DNA fragment I is amplified by using primers ilvG pro-catup / ilvG pro-catdown, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval020 by an electrotransformation method, and then the DNA fragment I is electrotransformed into the *Escherichia coli* Sval020 with the pKD46.

Electrotransformation conditions and steps are the same as the first step method for the *mgsA* gene knockout described in Example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers ilvG pro-YZup / ilvG p-YZdown, and a correct PCR product should be 2993 bp. A correct single colony is picked, and named as Sval0121.

In a second step, a genomic DNA of M1-93 is used as a template, and 188 bp of a DNA fragment II is amplified by using primers ilvG pro-up/ilvG pro-down. The DNA fragment II is used for the second homologous recombination. The DNA fragment II is electrotransformed into the strain Sval021.

Electrotransformation conditions and steps are the same as the second step method for the *mgsA* gene knockout described in Example 1. colonies were PCR verified using primers ilvG pro-YZup / ilvG p-YZdown and sequenced, and a correct colony amplification product is 462 bp. A correct single colony is picked, and named as Sval022.

### Example 12: Mutation of acetolactate synthase gene ilvH

A mutation is transferred into the ***ilvH*** gene so as to release feedback inhibition of L-valine through a two-step homologous recombination method. Specific steps are as follows.

In a first step, a pXZ-CS plasmid DNA is used as a template, 2719 bp of a DNA fragment I is amplified by using primers ilvH*-cat-up/ ilvH*-cat-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval022 by an electrotransformation method, and then the DNA fragment I is electrotransformed into the *Escherichia coli* Sval022 with the pKD46.

Electrotransformation conditions and steps are the same as the first step method for the *mgsA* gene knockout described in Example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers ilvH*-mutYZ-up/ ilvH*-mut-down, and a correct PCR product should be 3165 bp. A correct single colony is picked, and named as Sval023.

In a second step, a DNA of wild-type *Escherichia coli* ATCC 8739 is used as a template, and 467 bp of a DNA fragment II is amplified by using primers ilvH*-mut-up / ilvH*-mut-down. The DNA fragment II is used for the second homologous recombination. The DNA fragment II is electrotransformed into the strain Sval023.

Electrotransformation conditions and steps are the same as the second step method for the *mgsA* gene knockout described in Example 1. colonies were PCR verified using primers ilvH*-mutYZ-up/ ilvH*-mut-down and sequenced, and a correct colony amplification product is 619 bp. A correct single colony is picked, and named as Sval024.

### Example 13: Fermentation and production of L-valine using recombinant strain

### Sval024

A seed culture medium is formed by the following components (a solvent is water):
Glucose 20 g/L, corn syrup dry powder 10 g/L, KH₂PO₄ 8.8 g/L, (NH₄)₂SO₄ 2.5 g/L, and MgSO₄ •7H₂O 2 g/L.

The fermentation culture medium is most the same as the seed culture medium, and a difference is only that the glucose concentration is 50 g/L.

Anaerobic fermentation of Sval024 includes the following steps:
(1) Seed culture: a fresh clone on an LB plate is inoculated into a test tube containing 4 ml of the seed culture medium, and shake-cultured overnight at 37°C and 250 rpm. Then, a culture is transferred to 250 ml of a triangular flask containing 30 ml of the seed culture medium according to an inoculum size of 2% (V/V), and seed culture solution is obtained by shake culture at 37°C and 250 rpm for 12 hours, and used for fermentation medium inoculation.
(2) Fermentation culture: a volume of the fermentation culture medium in 500 ml of an fermenter is 250 ml, and the seed culture solution is inoculated into the fermentation culture medium according to an inoculum size of final concentration OD550=0.1, and fermented at 37°C and 150 rpm for 6 days, to obtain fermentation solution. The neutralizer is 5M ammonia, the pH was controlled at 7.0. No air was sparged during the fermentation.

Analytical method: an Agilent (Agilent-1260) high performance liquid chromatograph is used to determine components in the fermentation solution after fermentation for 6 days. The concentrations of glucose and organic acid in the fermentation solution are determined by using an Aminex HPX-87H organic acid analytical column of Biorad Company. A Sielc amino acid analysis column primesep 100 250x4.6mm is used for amino acid determination.

It is discovered from results that: strain Sval024 could produce 1.3 g/L of L-valine (an L-valine peak corresponding to a position in Fig. 2 appears) with a yield of 0.31 mol/mol after after 4 days fermentation under anaerobic conditions.

### Example 14: Cloning and integration of leucine dehydrogenase encoding gene leuDH

Referring to the reported(Ohshima, T. et.al, Properties of crystalline leucine dehydrogenase from Bacillus sphaericus. The Journal of biological chemistry 253, 5719-5725 (1978)) sequence of a leuDH from *Lysinibacillus sphaericus* IFO 3525, a leuDH gene was codon optimized and chemically synthesized (an optimized sequence is as shown in a sequence number 79).

. During the synthesis, an M1-93 artificial regulatory element is added before the *leuDH* gene to initiate expression of the *leuDH* gene, and inserted into a pUC57 vector to construct a plasmid pUC57-M1-93-leuDH (gene synthesis and vector construction are completed by Nanjing Genscript Biotechnology Co., Ltd.). The M1-93 artificial regulatory element and the *leuDH* gene are integrated into the fumarate reductase encoding gene *frd* site in strain Sval024 through a two-step homologous recombination method and substitute the *frd* gene, namely the *frd* gene is knocked out while the *leuDH* is integrated. Specific steps are as follows.

In a first step, a pXZ-CS plasmid DNA is used as a template, 2719 bp of a DNA fragment I is amplified by using primers frd-cs-up / frd-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval024 by an electrotransformation method, and then the DNA fragment I is electrotransformed into the *Escherichia coli* Sval024 with the pKD46.

Electrotransformation conditions and steps are the same as the first step method for the *mgsA* gene knockout described in Example 1. 200 µl of bacterial solution is spreaded onto an LB plate containing ampicillin (a final concentration is 100 µg/ml) and chloramphenicol (a final concentration is 34 µg/ml). After being cultured overnight at 30°C, colonies were PCR verified using primers XZ-frd-up/XZ-frd-down, and a correct PCR product should be 3493 bp. A correct single colony is picked, and named as Sval025.

In a second step, a pUC57-M1-93-IeuDH plasmid DNA is used as a template, and 1283 bp of a DNA fragment II is amplified by using primers frd-M93-up/frd-leuDH-down. The DNA fragment II is used for the second homologous recombination. The DNA fragment II is electrotransformed into the strain Sval025.

Electrotransformation conditions and steps are the same as the second step method for the *mgsA* gene knockout described in Example 1. Colonies were PCR verified using XZ-frd-up/XZ-frd-down and sequenced, and a correct colony amplification product is 2057 bp. A correct single colony is picked, and named as Sval026.

### Example 15: Fermentation and production of L-valine using recombinant strain Sval026

Components and preparation of seed culture medium and fermentation culture medium are the same as those described in Example 13.

The fermentation is performed in 500 mL of a fermentation vessel, and a fermentation process and an analysis process are the same as the fermentation process and the analysis process of the Sval024 described in Example 13.

It is discovered from results that: the strain Sval026 could produce 1.8 g/L of L-valine (an L-valine peak corresponding to a position in Fig. 2 appears) 0.56 mol/mol after 4 days fermentation under anaerobic conditions.

### Example 16: Regulation of transhydrogenase encoding gene pntAB using artificial regulatory element

Started from a strain Sval026, and an artificial regulatory element is used to regulate expression of a *pntAB* gene so as to activate activity of the transhydrogenase *PntAB.* Specific steps are as follows.

In a first step, a pXZ-CS plasmid DNA is used as a template, 2719 bp of a DNA fragment I is amplified by using primers pntAB-cs-up / pntAB-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval026 by an electrotransformation method, and then the DNA fragment I is electrotransformed into the *Escherichia coli* Sval026 with the pKD46.

Electrotransformation conditions and steps are the same as the first step method for the *mgsA* gene knockout described in Example 1. 200 µl of bacterial culture is spreaded onto an LB plate containing ampicillin (a final concentration is 100 µg/ml) and chloramphenicol (a final concentration is 34 µg/ml). After being cultured overnight at 30°C, colonies were PCR verified using primers pntAB-YZ-up/pntAB-YZ-down, and a correct PCR product should be 3459 bp. A correct single colony is picked, and named as Sval027.

In a second step, a genomic DNA of M1-93 is used as a template, and 188 bp of a DNA fragment II is amplified by using primers pntAB-P-up / pntAB-M93-down. The DNA fragment II is used for the second homologous recombination. The DNA fragment II is electrotransformed into the strain Sval027.

Electrotransformation conditions and steps are the same as the second step method for the *mgsA* gene knockout described in Example 1. Colonies were PCR verified using primers pntAB-YZ-up / pntAB-YZ-down, and a correct colony amplification product is 928 bp. A correct single colony is picked, and named as Sval028 (Table 1).

### Example 17: Regulation of NAD kinase encoding gene yfjB using artificial regulatory element

Started from a strain Sval028, and an artificial regulatory element is used to regulate expression of a NAD kinase gene *yfjB* so as to activate activity of the NAD kinase *YfjB,* so that the transhydrogenase and the NAD kinase may achieve a cofactor supply balance together. Specific steps are as follows.

In a first step, a pXZ-CS plasmid DNA is used as a template, 2719 bp of a DNA fragment I is amplified by using primers yfjb-cs-up / yfjb-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval028 by an electrotransformation method, and then the DNA fragment I is electrotransformed into the *Escherichia coli* Sval028 with the pKD46.

Electrotransformation conditions and steps are the same as the first step method for the *mgsA* gene knockout described in Example 1. 200 µl of bacterial culture is spread onto an LB plate containing ampicillin (a final concentration is 100 µg/ml) and chloramphenicol (a final concentration is 34 µg/ml). After being cultured overnight at 30°C, colonies were PCR verified using primers yfjb-YZ-up /yfjb-YZ-down, and a correct PCR product should be 3542 bp. A correct single colony is picked, and named as Sval029.

In a second step, a genomic DNA of M1-37 (Lu, et al., Appl Microbiol Biotechnol, 2012,93:2455-2462) is used as a template, and 188 bp of a DNA fragment II is amplified by using primers yfjb-P-up/ yfjb-M37-down. The DNA fragment II is used for the second homologous recombination. The DNA fragment II is electrotransformed into the strain Sval029.

Electrotransformation conditions and steps are the same as the second step method for the *mgsA* gene knockout described in Example 1. Colonies were PCR verified using primers yfjb-YZ-up/ yfjb-YZ-down, and a correct colony amplification product is 1011 bp. A correct single colony is picked, and named as Sval030 (Table 1).

### Example 18: Production of L-valine using recombinant strain Sval030

Components and preparation of seed culture medium and fermentation culture medium are the same as those described in Example 13.

The fermentation is performed in 500 mL fermentation vessel, a fermentation process and an analysis process are the same as the fermentation process and the analysis process of the Sval024 described in Example 13.

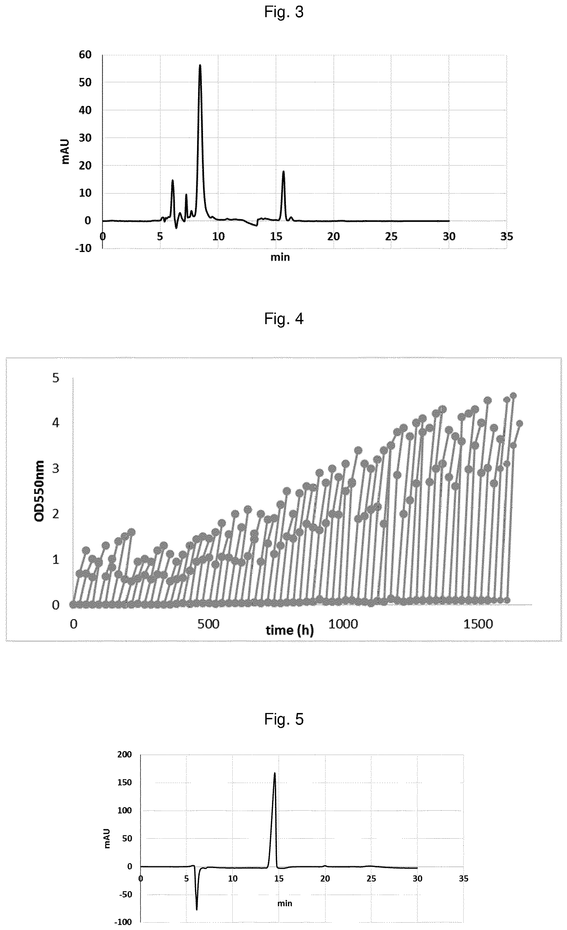

It is discovered from results that: Strain Sval030could produce 2.2 g/L of L-valine (an L-valine peak corresponding to a position in Fig. 2 appears) with a yield of 0.83 mol/mol after fermentation for 4 days under anaerobic conditions, Fig. 2 is a spectrum of the L-valine standard substance, and Fig. 3 is a spectrum of Sval030 fermentation solution.

### Example 19: Construction of recombinant strain Sval031

Started from Sval030, cell growth and L-valine production capacity are synchronously improved through metabolic evolution.

Metabolic evolution was carried out in 500 mL fermentation vessel with 250 mL fermentation culture medium. The fermentative pH was controlled at 7.0 by using 5 Mammonia as neutralizer. Components and preparation method of fermentation culture medium used for the metabolic evolution are the same as those of the fermentation culture medium described in Example 16. Every 24 hours, fermentation solution is transferred into a new fermentation vessel and the initial OD550 is 0.1. After 70 generations of the evolution, a strain Sval031 is obtained (Fig. 4). The strain Sval031 is preserved in the China General Microbiological Culture Collection Center (CGMCC) with the deposit number CGMCC 19456.

### Example 20: Fermentation of strain Sval031 to produce L-valine in 500 mL fermentation vessel

Components and preparation of a seed culture medium are the same as those described in Example 13.

The fermentation is performed in 500 mL fermentation vessel, and a fermentation culture medium is 250 ml. The fermentation culture medium is basically the same as the seed culture medium. A difference is that a glucose concentration is 100 g/L, and a neutralizer used is 5M ammonia, so that the fermentative pH is controlled in 7.0.

It is discovered from results that: after fermented in 500 mL fermentation vessel for 48 hours, strain Sval031 produced 53 g/L L-valine with a yield of 0.92 mol/mol, and impurities such as a heteroacid are not generated.

### Example 21: Production of L-valine by fermentation of recombinant strain Sval031 in 5 L fermentation vessel

Components, preparation and analytical method of a seed culture medium are the same as those described in Example 13. A fermentation culture medium is basically the same as the seed culture medium, and a difference is that a glucose concentration is 140 g/L.

The fermentation is performed in 5 L of a fermentation vessel (Shanghai Baoxing, BIOTECH-5BG) under anaerobic conditions, including the following steps:
(1) Seed culture: the seed culture medium in 500 ml of a triangular flask is 150 ml, and it is sterilized at 115°C for 15 min. After cooling, recombinant *Escherichia coli* Sval031 are inoculated into the seed culture medium according to an inoculum size of 1% (V/V), and cultured at 37°C and 100 rpm for 12 hours to obtain seed solution for inoculation of the fermentation culture medium.
(2) Fermentation culture: a volume of the fermentation culture medium in 5 L is 3 L, and it is sterilized at 115°C for 25 min. The seed solution is inoculated into the fermentation culture medium according to an inoculum size of final concentration OD550=0.2, and cultured under anaerobic conditions at 37°C for 48 days, and a stirring speed is 200 rpm, fermentation solution is obtained. The fermentation solution is all of substances in the fermentation vessel. No air was sparged during the fermentation.

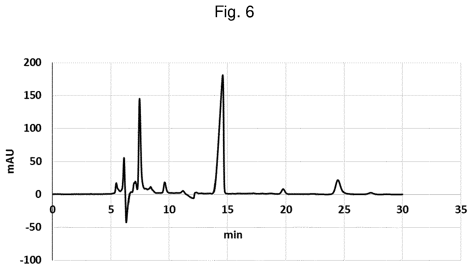

It is discovered from results that: after fermented in 5 L of the fermentation vessel for 48 hours, strain Sval031 produced 82 g/L L-valine with a yield of 0.93 mol/mol, and impurities such as a heteroacid are not generated. Fig. 5 is a spectrum of the L-valine standard substance, and Fig. 6 is a spectrum of Sval031 fermentation solution.

## Claims

1. A construction method of a recombinant microorganism for producing L-valine, comprising:
transferring an amino acid dehydrogenase gene into a microorganism, and/or activating activity of a transhydrogenase in the microorganism, and/or activating activity of a NAD kinase in the microorganism, so that enhancing the activity of the transhydrogenase and/or the NAD kinase in the microorganism;
optionally, the construction method further comprises transferring an acetohydroxy acid reductoisomerase encoding gene into the microorganism so as to enhance activity of an acetohydroxy acid reductoisomerase; the acetohydroxy acid reductoisomerase encoding gene is preferably an *ilvC* gene;
preferably, the amino acid dehydrogenase gene is NADH-dependent;
preferably, the amino acid dehydrogenase gene is a leucine dehydrogenase gene; and
more preferably, the amino acid dehydrogenase gene is leuDH, the transhydrogenase is PntAB, and the NAD kinase is YfjB.

2. The construction method according to claim 1, wherein the method further comprises one or more of the following modifications (1)-(7) to the recombinant microorganism according to claim 1:
(1) knocking out a gene *mgsA;*
(2) knocking out a gene *ldhA;*
(3) knocking out genes *pta* and/or *ackA;*
(4) knocking out genes *tdcD* and/or *tdcE;*
(5) knocking out a gene *adhE;*
(6) knocking out genes *frd* and/or *pflB;* and
(7) enhancing activity of AHAS and/or *ilvD;*
preferably, the AHAS is ilvBN, or ilvGM, or ilvlH; optionally, the activity of the ilvlH is enhanced by releasing feedback inhibition of valine to the ilvH, preferably, the *ilvH* gene is enhanced by mutation;
preferably, the above item (7) is selected for modification;
preferably, the above items (7) and (2) are selected for modification;
preferably, the above items (7) and (6) are selected for modification;
preferably, the above items (7), (2) and (5) are selected for modification;
preferably, the above items (7), (2) and (6) are selected for modification;
preferably, the above items (7), (1), and (3)-(6) are selected for modification;
preferably, the above items (1)-(7) are selected for modification;
preferably, the item (6) is achieved by substituting the *pflB* gene of the microorganism itself with the *ilvD* gene;
preferably, the item (6) is achieved by substituting the *frd* gene of the microorganism itself with the *leuDH* gene; and
preferably, the item (1) is achieved by substituting the *mgsA* gene of the microorganism itself with the *ilvC* gene.

3. The construction method according to claim 1 or 2, wherein the microorganism is *Escherichia coli;* and more preferably, the microorganism is *Escherichia coli* ATCC 8739.

4. The construction method according to any one of claims 1 to 3, wherein at least one regulatory element is used to activate or enhance activity of an encoding gene of the enzyme;
preferably, the regulatory element is selected from an M1-46 artificial regulatory element, an M1-93 artificial regulatory element or an M1-37 artificial regulatory element;
preferably, the M1-93 artificial regulatory element regulates encoding genes *pntAB, ilvD, leuDH, ilvBN* and *ilvGM;*
the M1-37 artificial regulatory element regulates an encoding gene *yfjB;* and
the M1-46 artificial regulatory element regulates an encoding gene *ilvC.*

5. The construction method according to any one of claims 1-4, wherein one or more copies of the enzyme encoding gene and the regulatory element are integrated into a genome of the microorganism, or a plasmid containing the enzyme encoding gene is transferred into the microorganism;
preferably, transfer, mutation, knockout, activation or regulation of the enzyme gene is completed by a method of integrating into the genome of the microorganism;
preferably, the transfer, mutation, knockout, activation or regulation of the enzyme gene is completed by a homologous recombination method; and
preferably, the transfer, mutation, knockout, activation or regulation of the enzyme gene is completed by a two-step homologous recombination method.

6. A recombinant microorganism obtained by the construction method according to any one of claims 1-5.

7. A method for acquiring a recombinant microorganism for highly producing L-valine, wherein it is obtained through metabolic evolution on the basis of the recombinant microorganism according to claim 6.

8. A recombinant microorganism, wherein the deposit number thereof is CGMCC 19456.

9. Use of the recombinant microorganism according to claim 6 or claim 8 in production of an L-valine.

10. A method for producing L-valine, wherein the method comprises: (1) fermenting the recombinant microorganism according to claim 6 or 8; and (2) separating and harvesting L-valine; preferably, the fermentation is carried out under anaerobic conditions.
